# EUROPÄISCHE PATENTSCHRIFT

(11) **EP 2 272 474 B1**
(45) Veröffentlichungstag und Bekanntmachung des Hinweises auf die Patenterteilung: **11.07.2012**
(21) Anmeldenummer: 10163372.5
(22) Anmeldetag: 20.05.2010
(51) Int. Cl.: A61F 9/04

(54) **Augenschutz-Kappe**
Eye protection cap
Capuchon de protection de l' oeil

(30) Priorität: 07.07.2009 DE 102009032178
(43) Veröffentlichungstag der Anmeldung: 12.01.2011
(73) Patentinhaber: Laservision GmbH & Co. KG, 90766 Fürth (DE)
(72) Erfinder: Bura, Peter, 73773, Aichwald (DE); Fröhlich, Thomas, 91207, Lauf a. d. Pegnitz (DE)
(74) Vertreter: Bauerschmidt, Peter

(56) Entgegenhaltungen:
- WO-A2-94/15557
- US-A- 4 570 626
- US-A- 4 655 767
- US-A1- 2008 148 461

## Beschreibung

Die Erfindung betrifft eine Augenschutz-Kappe zum Schutz eines Auges einer zur behandelnden Person vor elektromagnetischer Strahlung, insbesondere vor Laserstrahlung, gemäß dem Oberbegriff des Anspruchs 1.

Durch medizinische Laserbehandlungen können bei Personen Falten oder Hautanomalien wie Altersflecken und Warzen behandelt werden. Dabei müssen die Augen der zu behandelnden Person und die Augen der behandelnden Person vor der auftretenden Laserstrahlung geschützt werden.

Zum Schutz der Augen der zu behandelnden Person kann beispielsweise eine bekannte Augapfelbrille herangezogen werden, die zwei Halbschalen zum Bedecken der Augen umfasst. Die Halbschalen sind über einen Nasensteg miteinander verbunden und werden über ein Kopfband an dem Kopf der zu behandelnden Person gehalten. Insbesondere bei Laserbehandlungen im Dermatologie- und Kosmetikbereich schränken das Kopfband und der Nasensteg die Zugängigkeit zu den Bereichen um das Auge ein. Eine Behandlung in diesen Bereichen kann nur erfolgen, wenn das Kopfband und/oder der Nasensteg abgenommen bzw. örtlich versetzt werden. Die zu behandelnde Person ist dann einer erhöhten Gefährdung durch den Laser ausgesetzt. Eine gattungsgemäße Augapfelbrille ist beispielsweise aus der DE 10 2005 058 888 B3 bekannt.

Aus der US 2008/0148461 A1, die den nächsten Stand der Technik bildet, ist eine Augenschutz-Kappe bekannt, die zum Schutz vor Laserstrahlung getragen werden kann. Die Augenschutz-Kappe umfasst einen Randkörper und eine mit diesem in Verbindung stehende Deckschicht. Der Randkörper ist bei Benutzung der Augenschutz-Kappe mit dem Gesicht eines Trägers verklebt. Dafür ist innenseitig an dem Randkörper Klebstoff vorgesehen, der anfangs von einer abziehbaren Schutzfolie bedeckt sein kann. Die Deckschicht kann eine flexible Polymerfolie sein, während der Randkörper aus Silikon bestehen kann. Nachteilig an dieser bekannten Augenschutz-Kappe ist, dass deren Schutz vor elektromagnetischer Strahlung oftmals nicht befriedigend ist.

Die US 4,570,626 offenbart einen schalenförmigen Augenschutz zum Schutz eines Auges eines Patienten vor energiereichem Licht während einer Operation. Der Augenschutz ist auf die Augenhornhaut des Patienten zu legen. Er kann aus seinem flexiblen Material wie Silikon bestehen und außenseitig einen Greifvorsprung aufweisen. In dem Augenschutz sind Lüftungsöffnungen vorgesehen, um ein Festsaugen des Augenschutzes an der Augenhornhaut zu verhindern. Das Tragen dieses Augenschutzes wird im Allgemeinen als unbequem empfunden. Die Schutzwirkung dieses Augenschutzes ist nicht sonderlich hoch.

Aus der WO 94/15557 A2 ist ein weiterer Laserschutz zum Schutz der Augen eines Trägers vor Laserstrahlen bekannt. Der Laserschutz kann innenseitig an einem Silikonschaum eine metallische Folie haben, die eine Aluminiumfolie sein kann. Auf der dem Träger zugewandten Seite weist die Folie eine Klebeschicht auf. Das Tragen dieses Laserschutzes ist unangenehm. Der Laserschutz bietet im Allgemeinen keinen ausreichenden Schutz vor Laserstrahlen.

Die US 4,655,767 offenbart eine Medikamenten-Abgabevorrichtung, die einen aus Silikon bestehenden Klebebereich zum Ankleben an die Haut einer zu behandelnden Person aufweist. Die Vorrichtung umfasst ferner einen Formkörper, in dessen Innenraum sich das abzugebende Medikament befindet. Der Formkörper kann aus einer Aluminiumfolie bestehen.

Ferner sind selbstklebende Augenpads bekannt, die auf das Auge der zu behandelnden Person zu kleben sind. Diese Augenpads ermöglichen eine gute Zugänglichkeit zu sämtlichen Bereichen um das Auge der zu behandelnden Person, da keine Kopfbänder und Nasenstege zur Fixierung vorgesehen sind. Die Laserschutzwirkung der Augenpads ist baulich bedingt sehr gering. Nachteilig ist auch, dass die Augenpads Wegwerfartikel sind.

Es werden auch oftmals Kontaktlinsen aus Metall zum Schutz der zu behandelnden Person eingesetzt. Diese bieten zwar einen äußerst hohen Laserschutz, sind aber sehr unangenehm für die zu behandelnde Person zu tragen. Diese Kontaktlinsen werden im Wesentlichen nur verwendet, wenn das Augenlid der zu behandelnden Person behandelt werden soll.

Der Erfindung liegt die Aufgabe zu Grunde, eine Augenschutz-Kappe bereitzustellen, die einerseits einen äußerst hohen Schutz vor elektromagnetischer Strahlung liefert und andererseits eine gute Zugänglichkeit für eine behandelnde Person zu Bereichen um das Auge einer zu behandelnden Person bietet. Ferner soll die Augenschutz-Kappe für die zu behandelnde Person bequem zu tragen sein.

Diese Aufgabe wird erfindungsgemäß durch die in dem Anspruch 1 angegebenen Merkmale gelöst. Der Kern der Erfindung liegt darin, dass die Augenschutz-Kappe einen hohlen Silikon-Formkörper aufweist, der an der zu behandelnden Person selbsthaltend angebracht werden kann. Separate Haltemittel wie Kopfbänder, Nasenstege oder dergleichen sind somit nicht erforderlich. Der Formkörper ist formstabil. Er kann aber auch in gewissem Maße flexibel bzw. in seiner Form veränderbar sein. Die Form des Formkörpers kann aber bei bestimmungsgemäßer Benutzung der Augenschutz-Kappe insbesondere nicht dauerhaft verändert werden.

Weitere vorteilhafte Ausgestaltungen sind in den Unteransprüchen angegeben.

Nachfolgend wird unter Bezugnahme auf die beigefügte Zeichnung eine bevorzugte Ausführungsform der Erfindung beschrieben. Dabei zeigen:
- Fig. 1: einen Kopf einer zu behandelnden Person, die zwei erfindungsge- mäße Augenschutz-Kappen trägt,

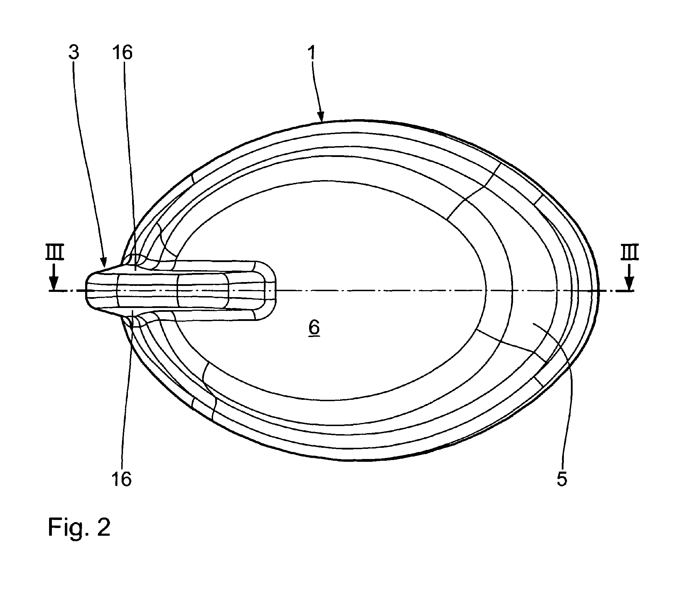

- Fig. 2: eine Draufsicht auf eine erfindungsgemäße, in Fig. 1 dargestellte Augenschutz-Kappe,

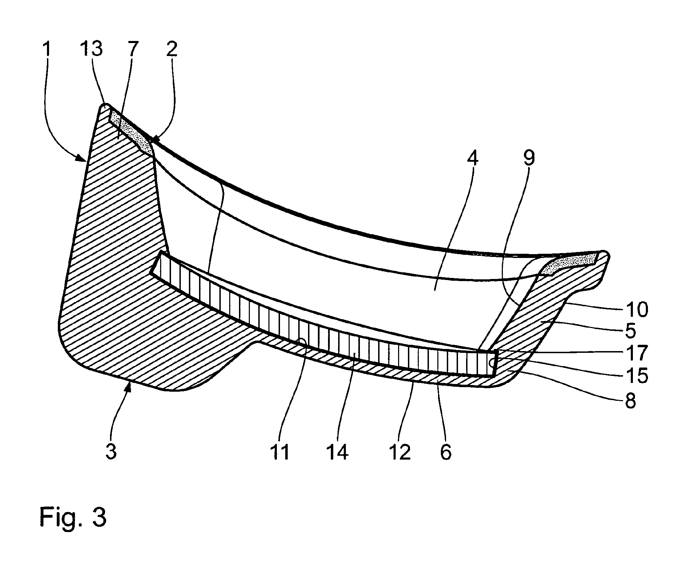

- Fig. 3: einen Schnitt durch die in Fig. 2 dargestellte Augenschutz-Kappe gemäß der Schnittlinie III-III, und

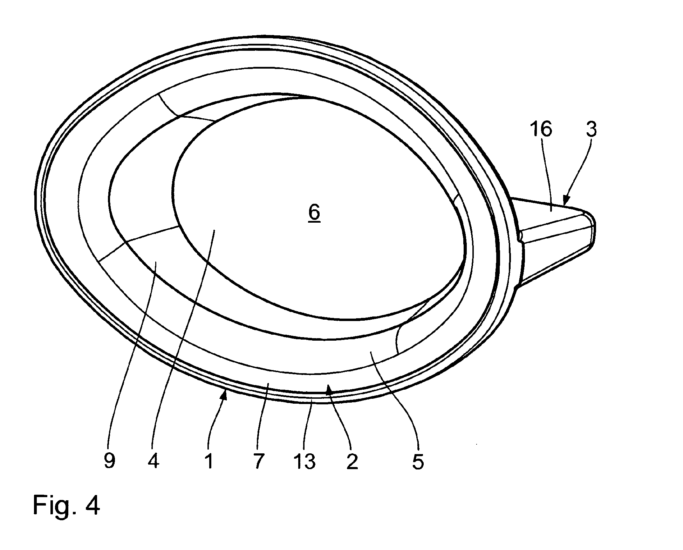

- Fig. 4: eine Ansicht, die eine in den Figuren 1 bis 3 dargestellte Augen- schutz-Kappe von hinten zeigt.

Eine Augenschutz-Kappe zum Schutz eines Auges einer zu behandelnden Person vor elektromagnetischer Strahlung, insbesondere vor Laserstrahlung, umfasst einen einstückigen, hohlen Formkörper 1, einen Selbsthaltebereich 2 zum eigenständigen Halten des Formkörpers 1 an der zu behandelnden Person und einen Handhabungsgriff 3. Der Formkörper 1 ist aus einem Silikonmaterial mit einer Härte von insbesondere mindestens 50 Shore A, und von vorzugsweise 60 Shore A, gebildet und begrenzt einen hohlen Formkörper-Innenraum 4 nach außen. Die Augenschutz-Kappe ist zum Einsatz bei medizinischen bzw. kosmetischen Laserbehandlungen vorgesehen.

Der Formkörper 1, der aus einem Silikonmaterial gebildet ist, ist schalenartig ausgebildet und weist eine gestreckte Grundform auf. Durch die gestreckte Grundform ist der Formkörper-Innenraum 4 im Querschnitt im Wesentlichen oval. Der Formkörper 1 hat eine umlaufende, geschlossene Seitenwandung 5 und eine mit der Seitenwandung 5 randseitig verbundene, im Wesentlichen ebene Decke 6, die bezogen auf den Formkörper-Innenraum 4 leicht konvex gekrümmt ist. Die Seitenwandung 5 verläuft quer zu der Decke 6. Die Seitenwandung 5 und die Decke 6 sind einstückig ausgebildet.

Die Seitenwandung 5 hat einen freien Fuß-Rand 7, der gegenüberliegend zu einem Kopf-Bereich 8 der Seitenwandung 5 ist und auch von der Seitenwandung 5 nach seitlich außen vorspringt. Der Fuß-Rand 7 erstreckt sich derart, dass er bei der Applikation der Augenschutz-Kappe ein Auge bzw. eine Augenhöhle der zu behandelnden Person umgibt. Bei dem Kopf-Bereich 8 der Seitenwandung 5, der beabstandet zu dem Fuß-Rand 7 ist, grenzt die Decke 6 an die Seitenwandung 5 an. Die Seitenwandung 5 hat eine im Wesentlichen konstante Höhe und verjüngt sich über ihre Höhe ausgehend von ihrem Fuß-Rand 7 in Richtung ihres Kopf-Bereichs 8, so dass sich letztendlich auch der Formkörper-Innenraum 4 von dem Fuß-Rand 7 der Seitenwandung 5 zu dem Kopf-Bereich 8 der Seitenwandung 5 gleichmäßig verjüngt. Der Formkörper-Innenraum 4 ist seitlich durch die Seitenwandung 5 und nach oben durch die Decke 6 begrenzt. Bei dem Fuß-Rand 7 ist der Formkörper-Innenraum 4 nach außen offen bzw. zugänglich.

Ferner weist die Seitenwandung 5 einen nach innen gewandten Innenbereich 9 und einen diesem gegenüberliegenden Außenbereich 10 auf. Die Decke 6 hat ebenfalls einen Innenbereich 11 und einen nach außen gewandten Außenbereich 12. Der Formkörper-Innenraum 4 wird durch den Innenbereich 9 der Seitenwandung 5 und durch den Innenbereich 11 der Decke 6 begrenzt.

An dem Fuß-Rand 7 ist der Selbsthaltebereich 2 vorgesehen, der auch aus einem Silikonmaterial besteht und schichtartig ausgestaltet ist. Der Selbsthaltebereich 2 erstreckt sich an dem Fuß-Rand 7 über den gesamten Umfang der Seitenwandung 5, so dass der Selbsthaltebereich 2 geschlossen umlaufend ist. Obwohl der Formkörper 1 und der Selbsthaltebereich 2 aus dem gleichen Basismaterial, nämlich insbesondere Silikon, bestehen, unterscheidet sich das Silikonmaterial des Selbsthaltebereichs 2 von dem Silikonmaterial des Formkörpers 1. Es ist wesentlich weicher als das Silikonmaterial des Formkörpers 1 und hat eine Härte von insbesondere höchstens 10 Shore A, vorzugsweise von in etwa 0 Shore A. Aufgrund des Aufbaus aus dem gleichen Basismaterial sind der Formkörper 1 und der Selbsthaltebereich 2 besonders gut und insbesondere innig miteinander verbunden. Der Selbsthaltebereich 2 ist außerdem sehr selbstklebend, so dass die Augenschutz-Kappe allein mittels Klebekraft an der Augenpartie des Patienten fixierbar ist. Der Selbsthaltebereich 2 ist nach seitlich außen durch einen Steg 13 begrenzt, der von der Seitenwandung 5 nach unten vorspringt. Die Höhe des Stegs 13 entspricht in etwa der Dicke des Selbsthaltebereichs 2. Der Selbsthaltebereich 2 zieht sich bis in den Formkörper-Innenraum 4 hinein, so dass der Selbsthaltebereich 2 zumindest bereichsweise über seine Breite gekrümmt verläuft.

In den Formkörper 1 ist innen ein Zusatzschutzelement 14 eingesetzt, das auch vor elektromagnetischer Strahlung, insbesondere vor Laserstrahlung, schützen kann. Es weist eine an die Decke 6 angepasste Krümmung auf, so dass es innenseitig über den gesamten Innenbereich 11 an der Decke 6 anliegt. Das Zusatzschutzelement 14 und die Decke 6 erstrecken sich im Wesentlichen in einander parallelen Flächen. Das Zusatzschutzelement 14 ist hier als ovales Metallplättchen ausgebildet und bildet quasi eine weitere Decke. Das Zusatzschutzelement 14 ist dicker als die Decke 6. Es ist starr ausgebildet. Insbesondere besteht das Metallplättchen aus Aluminium. Andere geeignete Materialien können ebenfalls Anwendung finden.

Zur örtlichen, aber auch zur wieder lösbaren Festlegung des Zusatzschutzelements 14 in dem Formkörper 1 ist eine nutartige Halteausnehmung 15 in dem Innenbereich 9 der Seitenwandung 5 in dessen Kopf-Bereich 8 vorgesehen, die umlaufend ausgebildet und zu dem Formkörper-Innenraum 4 hin offen ist. Die Höhe der Halteausnehmung 15 entspricht in etwa der Dicke des Zusatzschutzelements 14. Das Zusatzschutzelement 14 kann über die Halteausnehmung 15 in dem Formkörper-Innenraum 4 an der Decke 6 anliegend örtlich festgelegt werden. Das Zusatzschutzelement 14 ist auch wieder herausnehmbar. Die Halteausnehmung 15 und das Zusatzschutzelement 14 sind in Form und Größe aneinander angepasst. Das Zusatzschutzelement 14 kann aber auch auf andere Art und Weise festgelegt werden. Beispielsweise kann es auch an der Decke 6 oder über mehrere Halteausnehmungen, Rastnasen oder über eine Klemmung oder mittels Haftreibung an dem Innenbereich 9 der Seitenwandung 5 fixiert werden.

Der Handhabungsgriff 3 ist einstückig mit dem Formkörper 1 verbunden und ist aus dem gleichen Silikonmaterial wie der Formkörper 1 gebildet. Er ist als Vorsprung ausgestaltet, der von dem Außenbereich 10 der Seitenwandung 5 nach seitlich außen und von dem Außenbereich 12 der Decke 6 nach oben vorspringt. Der Handhabungsgriff 3 weist zwei einander gegenüberliegende Griffflächen 16 auf. Die Augenschutz-Kappe ist bezüglich einer Symmetrieebene symmetrisch, die mittig durch den Handhabungsgriff 3 geht.

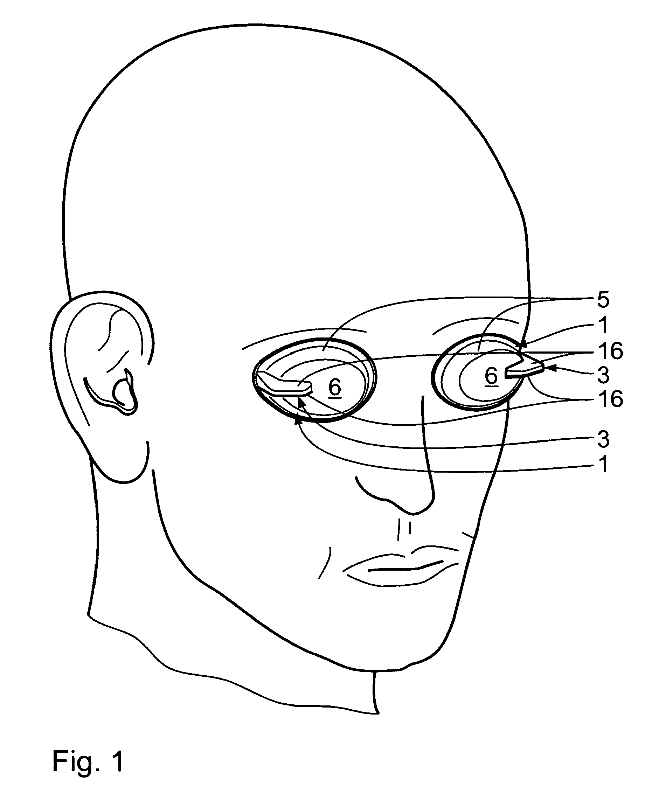

Nachfolgend werden die Platzierung und der Einsatz der Augenschutz-Kappe detaillierter beschrieben. Wie aus Fig. 1 ersichtlich ist, bedecken die beiden Augenschutz-Kappen die zwei Augen der zu behandelnden Person vollständig. Es ist nur der Kopf der zu behandelnden Person dargestellt. Für jedes Auge ist eine eigene Augenschutz-Kappe vorgesehen. Nachdem die Augenschutz-Kappen identisch ausgebildet sind, wird nun nur auf eine Augenschutz-Kappe eingegangen. Der Selbsthaltebereich 2 der Augenschutz-Kappe läuft um das Auge bzw. die Augenhöhle der zu behandelnden Person und liegt an den zu dem Auge benachbarten Gesichtsbereichen der zu behandelnden Person dicht an. Durch die Klebewirkung des Selbsthaltebereichs 2 wird der Formkörper 1 dort sicher ohne weitere Hilfsmittel gehalten. Aufgrund der nachgiebigen Ausgestaltung des Selbsthaltebereichs 2 und des auch in gewissem Maße verformbaren Formkörpers 1 kann sich die Augenschutz-Kappe an die jeweilige zu behandelnde Person exakt anpassen und dicht an dem Gesicht der zu behandelnden Person anliegen. Anatomische Unterschiede bei den zu behandelnden Personen können dadurch ausgeglichen werden. Der Formkörper-Innenraum 4 befindet sich direkt über dem Auge der zu behandelnden Person. Er ist zu dem Auge der zu behandelnden Person offen. Durch die Seitenwandung 5 verläuft die Decke 6 beabstandet zu dem Auge der zu behandelnden Person, so dass die zu behandelnde Person ihr bedecktes Auge auch während der Behandlung öffnen und schließen kann. Die Wimpern der zu behandelnden Person stoßen nicht an der Augenschutz-Kappe an. Dies erhöht den Tragekomfort.

Die Augenschutz-Kappe kann durch den Handhabungsgriff 3 von der zu behandelnden Person abgenommen bzw. auf das Auge der zu behandelnden Person aufgesetzt werden. Dabei ist die Augenschutz-Kappe an den Griffflächen 16 zu halten.

In den Formkörper 1 kann vor dem Einsatz von dem Fuß-Rand 7 her das Zusatzschutzelement 14 eingefügt werden. Es ist von unten manuell in die Halteausnehmung 15 zu drücken, in die es dann einspringt und sicher gehalten wird. In Richtung auf den Fuß-Rand 7 ist das Zusatzschutzelement 14 durch einen stegartigen Halte-Ansatz 17 gesichert, der von dem Innenbereich 9 der Seitenwandung 5 nach innen in den Formkörper-Innenraum 4 vorspringt und sich über den gesamten Umfang der Seitenwandung 5 erstreckt. Der Halte-Ansatz 17 ist einstückig mit dem Formkörper 1 verbunden und untergreift das Zusatzschutzelement 14 randseitig. Beim Einfügen des Zusatzschutzelements 14 ist das Zusatzschutzelement 14 über den Halte-Ansatz 17 zu drücken. Durch das Zusatzschutzelement 14 kann der Schutz vor Laserstrahlung weiter erhöht werden.

Die Augenschutz-Kappe und gegebenenfalls auch das Zusatzschutzelement 14 verhindern sicher, dass Laserstrahlung bei einer Laserbehandlung in den Formkörper-Innenraum 4 bzw. zu dem bedeckten Auge der zu behandelnden Person gelangen kann. Auch von dem Fuß-Rand 7 kann keine Laserstrahlung in den Formkörper-Innenraum 4 bzw. zu dem bedeckten Auge der zu behandelnden Person kommen.

Aufgrund der hohen Klebewirkung des Selbsthaltebereichs 2 sind keine zusätzlichen Haltemittel erforderlich, wie z. B. Nasenstege, die sonst herkömmliche Augenschutz-Kappen miteinander verbinden. Auch andere Hilfsmittel, die die Augenschutz-Kappen an dem Kopf des Trägers halten, erübrigen sich. Nach dem Stand der Technik werden hierfür üblicherweise Bügel oder Kopfbänder herangezogen. Die behandelnde Person, wie ein Arzt oder eine Kosmetikerin, kann somit sämtliche Gesichtsbereiche einfach und problemlos erreichen, die unmittelbar benachbart zu dem Fuß-Rand 7 sind.

Die Augenschutz-Kappe ist z. B. durch Dampf sterilisierbar. Sie ist somit wieder verwendbar. Die Augenschutz-Kappe ist außerdem thermisch äußerst beständig, was auf die Silikonausführung des Formkörpers 1 und des Selbsthaltebereichs 2 zurückzuführen ist.

## Patentansprüche

1. Augenschutz-Kappe zum Schutz eines Auges einer zu behandelnden Person vor elektromagnetischer Strahlung, wobei die Augenschutz-Kappe einen formstabilen Formkörper (1) umfasst, der
a) einen freien Rand (7) zur benachbarten Anordnung zu der zu behandelnden Person aufweist,
b) einen hohlen Formkörper-Innenraum (4) begrenzt,
c) aus einem Silikonmaterial gebildet ist, und
d) einen Selbsthaltebereich (2) zum eigenständigen Halten an der zu behandelnden Person aufweist,
**dadurch gekennzeichnet, dass**
e) in dem Formkörper (1) ein Zusatzschutzelement (14) angeordnet ist,
f) wobei in dem Formkörper (1) mindestens eine Halteausnehmung (15) zur Fixierung des einsetzbaren Zusatzschutzelements (14) vorgesehen ist.

2. Augenschutz-Kappe nach Anspruch 1, **dadurch gekennzeichnet, dass** der Formkörper (1) eine Seitenwandung (5) und eine mit der Seitenwandung (5) in Verbindung stehende Decke (6) umfasst, wobei die Seitenwandung (5) den freien Rand (7) aufweist.

3. Augenschutz-Kappe nach Anspruch 1 oder 2, **dadurch gekennzeichnet, dass** der Selbsthaltebereich (2) an dem freien Rand (7) der Seitenwandung (5) vorgesehen ist.

4. Augenschutz-Kappe nach einem der vorherigen Ansprüche, **dadurch gekennzeichnet, dass** der Selbsthaltebereich (2) selbstklebend ist.

5. Augenschutz-Kappe nach einem der vorherigen Ansprüche, **dadurch gekennzeichnet, dass** der Selbsthaltebereich (2) durch ein flexibles Silikonmaterial gebildet ist.

6. Augenschutz-Kappe nach Anspruch 5, **dadurch gekennzeichnet, dass** das Silikonmaterial des Selbsthaltebereichs (2) weicher als das Silikonmaterial des Formkörpers (1) ist.

7. Augenschutz-Kappe nach einem der vorherigen Ansprüche, **dadurch gekennzeichnet, dass** der Selbsthaltebereich (2) geschlossen umlaufend ist.

8. Augenschutz-Kappe nach einem der vorherigen Ansprüche, **dadurch gekennzeichnet, dass** das Zusatzschutzelement (14) an der Decke (6) anliegt.

9. Augenschutz-Kappe nach Anspruch 8, **dadurch gekennzeichnet, dass** sich das Zusatzschutzelement (14) über mindestens einen Großteil der Decke (6) erstreckt.

10. Augenschutz-Kappe nach einem der Ansprüche 1, 8 oder 9, **dadurch gekennzeichnet, dass** das Zusatzschutzelement (14) lösbar in den Formkörper-Innenraum (4) eingesetzt ist.

11. Augenschutz-Kappe nach einem der vorherigen Ansprüche, **dadurch gekennzeichnet, dass** die mindestens eine Halteausnehmung (15) in der Seitenwandung (5) benachbart zu der Decke (6) vorgesehen ist.

12. Augenschutz-Kappe nach einem der Ansprüche 1 oder 8 bis 11, **dadurch gekennzeichnet, dass** das Zusatzschutzelement (14) an dem Formkörper (1) über die mindestens eine Halteausnehmung (15) befestigt ist.

13. Augenschutz-Kappe nach einem der vorherigen Ansprüche, **gekennzeichnet durch** einen Handhabungsgriff (3), der an dem Formkörper (1) angebracht ist.

14. Augenschutz-Kappe nach Anspruch 1, **dadurch gekennzeichnet, dass** das Zusatzschutzelement (14) in Form eines Metallplättchens vorliegt.

## Claims

1. Eye protection cap to protect an eye of a person to be treated against electromagnetic radiation, wherein the eye protection cap comprises a dimensionally stable shaped body (1), which
a) has a free edge (7) for adjacent arrangement in relation to the person to be treated,
b) limits a hollow shaped body interior (4),
c) is formed from a silicone material, and
d) has a self-holding region (2) for independent holding on the person to be treated
**characterized in that**
e) an additional protection element (14) is arranged in the shaped body (1),
f) wherein at least one holding recess (15) for fixing the insertable additional protection element (14) is provided in the shaped body (1).

2. Eye protection cap according to claim 1, **characterised in that** the shaped body (1) comprises a side wall (5) and a cover (6) connected to the side wall (5), the side wall (5) having the free edge (7).

3. Eye protection cap according to claim 1 or 2, **characterised in that** the self-holding region (2) is provided on the free edge (7) of the side wall (5).

4. Eye protection cap according to any one of the preceding claims, **characterised in that** the self-holding region (2) is self-adhesive.

5. Eye protection cap according to any one of the preceding claims, **characterised in that** the self-holding region (2) is formed by a flexible silicone material.

6. Eye protection cap according to claim 5, **characterised in that** the silicone material of the self-holding region (2) is softer than the silicone material of the shaped body (1).

7. Eye protection cap according to any one of the preceding claims, **characterised in that** the self-holding region (2) is peripherally closed.

8. Eye protection cap according to any one of the preceding claims, **characterised in that** the additional protection element (14) abuts on the cover (6).

9. Eye protection cap according to claim 8, **characterised in that** the additional protection element (14) extends over at least a large part of the cover (6).

10. Eye protection cap according to any one of claims 1, 8 or 9, **characterised in that** the additional protection element (14) is releasably inserted in the shaped body interior (4).

11. Eye protection cap according to any one of the preceding claims, **characterised in that** the at least one holding recess (15) is provided in the side wall (5) adjacent to the cover (6).

12. Eye protection cap according to any one of claims 1 or 8 to 11, **characterised in that** the additional protection element (14) is fastened to the shaped body (1) by means of the at least one holding recess (15).

13. Eye protection cap according to any one of the preceding claims, **characterised by** a handling handle (3) which is attached to the shaped body (1).

14. Eye protection cap according to claim 1, **characterized in that** the additional protection element (14) is in the form of a small metal plate.

## Revendications

1. Capuchon de protection de l'oeil d'une personne à traiter contre un rayonnement électromagnétique, le capuchon de protection de l'oeil comprenant un corps moulé (1) indéformable qui
a) présente un rebord libre (7) pour la mise en place immédiate sur la personne à traiter,
b) délimite un espace interne creux du corps moulé (4),
c) est formé dans un matériau silicone, et
d) présente une zone restant en place toute seule (2) pour un maintien autonome sur la personne à traiter,
**caractérisé en ce**
e) **qu'**un élément de protection complémentaire (14) est disposé dans le corps moulé (1),
f) où au moins une cavité de maintien (15) pour la fixation de l'élément de protection complémentaire (14), pouvant être inséré, est prévue dans le corps moulé (1).

2. Capuchon de protection de l'oeil selon la revendication 1 **caractérisé en ce que** le corps moulé (1) comprend une paroi latérale (5) et un couvercle (6) étant relié avec la paroi latérale (5), la paroi latérale présentant le rebord libre (7).

3. Capuchon de protection de l'oeil selon les revendications 1 ou 2 **caractérisé en ce que** la zone restant en place toute seule (2) est prévue sur le rebord libre (7) de la paroi latérale (5).

4. Capuchon de protection de l'oeil selon l'une des revendications précédentes **caractérisé en ce que** la zone restant en place toute seule (2) est autoadhésive.

5. Capuchon de protection de l'oeil selon l'une des revendications précédentes **caractérisé en ce que** la zone restant en place toute seule (2) est formée dans un matériau silicone flexible.

6. Capuchon de protection de l'oeil selon la revendication 5 **caractérisé en ce que** le matériau silicone de la zone restant en place toute seule (2) est plus souple que le matériau silicone du corps moulé (1).

7. Capuchon de protection de l'oeil selon l'une des revendications précédentes **caractérisé en ce que** la zone restant en place toute seule (2) est fermée sur son pourtour.

8. Capuchon de protection de l'oeil selon l'une des revendications précédentes **caractérisé en ce que** l'élément de protection complémentaire (14) est plaqué contre le couvercle (6).

9. Capuchon de protection de l'oeil selon la revendication 8 **caractérisé en ce que** l'élément de protection complémentaire (14) s'étend sur au moins une grande partie du couvercle (6).

10. Capuchon de protection de l'oeil selon l'une des revendications 1, 8 ou 9 **caractérisé en ce que** l'élément de protection complémentaire (14) est inséré, pouvant être détaché, dans l'espace interne du corps moulé (4).

11. Capuchon de protection de l'oeil selon l'une des revendications précédentes **caractérisé en ce qu'**au moins une cavité de maintien (15) est prévue dans la paroi latérale (5) au voisinage du couvercle (6).

12. Capuchon de protection de l'oeil selon l'une des revendications 1 ou de 8 à 11 **caractérisé en ce que** l'élément de protection complémentaire (14) sur le corps moulé (1) est fixé au dessus d'au moins une cavité de maintien (15).

13. Capuchon de protection de l'oeil selon l'une des revendications précédentes **caractérisé par** une poignée de manipulation (3) qui est rapportée sur le corps moulé (1).

14. Capuchon de protection de l'oeil selon la revendication 1 **caractérisé en ce que** l'élément de protection complémentaire (14) est présent sous la forme d'une plaquette métallique.
